(19) Europäisches Patentamt
European Patent Office
Office européen des brevets

(11) Veröffentlichungsnummer : **0 063 731**
**B1**

(12) **EUROPÄISCHE PATENTSCHRIFT**

(45) Veröffentlichungstag der Patentschrift : **04.07.84**

(21) Anmeldenummer : **82103013.7**

(22) Anmeldetag : **08.04.82**

(51) Int. Cl.³ : **C 07 C 67/46**, C 07 C 69/612, C 07 C 69/712, C 07 C 69/708, C 07 C 69/635, C 07 C 51/09, C 07 C 57/30, C 07 C 59/68, C 07 C 59/64, C 07 C 57/58

(54) **Verfahren zur Herstellung von optisch aktiven Carbonsäuren.**

(30) Priorität : **25.04.81 DE 3116474**

(43) Veröffentlichungstag der Anmeldung : **03.11.82 Patentblatt 82/44**

(45) Bekanntmachung des Hinweises auf die Patenterteilung : **04.07.84 Patentblatt 84/27**

(84) Benannte Vertragsstaaten : **AT BE CH DE FR GB IT LI NL SE**

(56) Entgegenhaltungen :
**J. LIEBIGS ANNALEN DER CHEMIE, Band 722, 1969, Seiten 1-11, Weinheim, DE. H. PRACEJUS et al.:"Tertiäre Amine mit einem asymmetrischen C-Atom als Katalysatoren für die asymetrische Synthese von alpha-Phenyl-propionsäure-methylester"**

(73) Patentinhaber : **BASF Aktiengesellschaft**
**Carl-Bosch-Strasse 38**
**D-6700 Ludwigshafen (DE)**

(72) Erfinder : **Ruechardt, Christoph, Dr.**
**Ringstrasse 18**
**D-7801 Stegen b. Freiburg (DE)**
Erfinder : **Jaehme, Joachim**
**Eschholzstrasse 35**
**D-7800 Freiburg (DE)**
Erfinder : **Salz, Ulrich**
**Rennweg 28**
**D-7800 Freiburg (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft ein verbessertes Verfahren zur Herstellung von optisch aktiven Carbonsäuren der allgemeinen Formel I

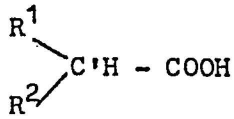
(I)

in welcher $R^1$ für einen organischen Rest steht, der über ein C-Atom mit dem asymmetrischen C'-Atom verknüpft ist und in welcher $R^2$ einen der Reste $R^1$ (jedoch nicht den gleichen Rest), Halogen oder einen organischen Rest bedeutet, der über ein O-Atom an das asymmetrische C'-Atom gebunden ist.

Aus der Arbeit von Pracejus (Liebigs Annalen der Chemie, Band 634 (1960), S. 9 ff) ist es bekannt, Methylphenylketen in Gegenwart eines optisch aktiven tertiären Amins wie Brucin mit einem achiralen Alkohol zu dem entsprechenden Carbonsäureester umzusetzen

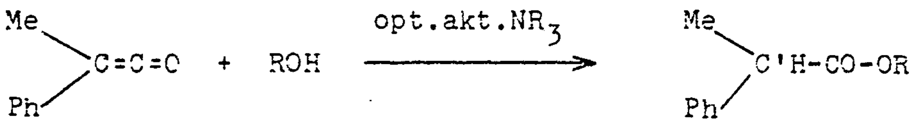


Me = Methyl
Ph = Phenyl

wobei einer der beiden optisch isomeren Ester bevorzugt entsteht. Die optische Ausbeute (= % des überwiegenden Isomeren minus % des anderen Isomeren) beträght hierbei jedoch nur 2-10 % entsprechend einem Isomerenverhältnis von 51 : 49 bis 55 : 45, sofern man nicht bei extrem tiefen Temperaturen arbeitet. Für technische Synthesen von optisch aktiven Carbonsäureestern oder von deren Folgeprodukten wie den freien Säuren kommt dieses Verfahren daher nicht in Betracht.

Weiterhin ist es aus der Arbeit von Anders et al (Angewandte Chemie, Band 85 (1973), S. 16 ff) bekannt, Phenyltrifluormethylketen in Abwesenheit eines Amins mit optisch aktiven sekundären Alkoholen in die entsprechenden Ester zu überführen. Die Untersuchung der hieraus durch Hydrolyse gewonnenen freien Carbonsäure des Typs I ($R^1$ = Phenyl, $R^2$ = Trifluormethyl) zeigte, daß sich jeweils eines der Isomeren entsprechend einer optischen Ausbeute von 22-36 % im Überschuß gebildet hatte.

Da aber auch diese Ergebnisse noch nicht befriedigen können, war es Aufgabe der Erfindung, die Carbonsäuren I auf wirtschaftlichere und verfahrenstechnisch einfachere Weise in höherer optischer Reinheit herzustellen als bisher.

Demgemäß wurde gefunden, daß man die eingangs definierten Carbonsäuren I durch Umsetzung eines Ketens II

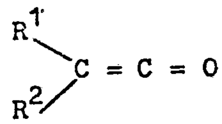
(II)

mit einem Alkohol in homogener flüssiger Phase in Gegenwart eines tertiären Amins III und anschließende Überführung des so erhaltenen Esters in die Säure in unerwartet hohen optischen Ausbeuten erhält, wenn man hierzu als Alkohol einen optisch aktiven Alkohol IV verwendet.

In einer vorteilhaften Abwandlung dieses Verfahrens kann man anstelle des Amins III und des Alkohols IV auch einen optisch aktiven tert.-Aminoalkohol verwenden, also eine Verbindung, welche die Funktionen von III und IV in sich vereinigt.

Nach den bisherigen Beobachtungen ist das gute Gelingen des erfindungsgemäßen Verfahrens von der chemischen Natur der Reste $R^1$ und $R^2$ in den Ausgangsverbindungen II weitgehend unabhängig, so daß diese Reste prinzipiell beliebig sein können. Im wesentlichen haben nur sterische Effekte einen Einfluß auf die optische Ausbeute, welche 30 % normalerweise jedoch nicht unterschreitet. Damit jedoch das C-Atom, welches diese Reste trägt, chiral werden kann, darf keiner dieser Reste Wasserstoff bedeuten und dürfen diese Reste nicht gleich sein.

Als Reste $R^1$ kommen in Betracht :

— aliphatische Reste mit 1-20 C-Atomen wie Alkyl-, Alkenyl- und Alkinylreste, vorzugsweise $C_1$-$C_4$-Alkylgruppen,

— cycloaliphatische Reste mit 5 und 6 Ringgliedern wie vorzugsweise Cyclopentyl-, Cyclopentenyl-, Cyclohexyl- und Cyclohexenylreste,

— araliphatische Reste wie vorzugsweise der Benzylrest,

— isocyclische und heterocyclische aromatische Reste wie vorzugsweise die Phenyl-, Naphthyl- und Pyridylgruppe,

wobei diese Reste ihrerseits inerte Substituenten wie $C_1$-$C_{10}$-Alkyl- und Alkenylgruppen, Halogen,

Cyangruppen, Nitrogruppen, Formylgruppen, $C_1$-$C_4$-Alkoxygruppen, $C_1$-$C_4$-Carbalkoxygruppen, $C_1$-$C_4$-Arylgruppen, $C_1$-$C_4$-Acyloxygruppen und substituierte Aminogruppen tragen können.

Als Reste $R^2$ kommen die Reste $R^1$ mit der Maßgabe in Betracht, daß $R^2 \neq R^1$ sein muß, und darüber hinaus Halogen wie Fluor, Chlor, Brom und Jod sowie organische Reste, die über ein Sauerstoffatom an das prochirale C-Atom gebunden sind. Ausgangsverbindungen II der letztgenannten Art haben somit die allgemeine Formel IIa

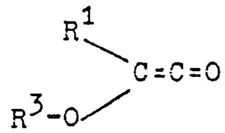

(IIa)

in welcher der Rest $R^3$ z. B. einer der für $R^1$ genannten Reste sein kann.

Die Ketene II sind entweder bekannt oder nach bekannten Methoden erhältlich, und zwar vorzugsweise durch Umsetzung der entsprechenden Carbonsäurechloride II' mit einem tertiären Amin

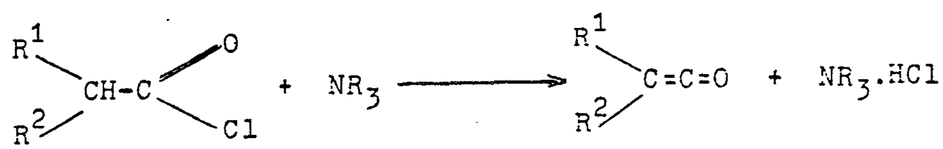

(II') (II)

Da es nicht erforderlich ist, die Ketene II vor der erfindungsgemäßen Umsetzung zu isolieren, ist es in der Praxis besonders vorteilhaft, hierfür die Reaktionsgemische einzusetzen, die bei der Herstellung der Ketene II *in situ,* z. B. aus den Carbonsäurechloriden II' anfallen, zumal man für beide Reaktionen auch das gleiche tertiäre Amin III verwenden kann.

Das für die erfindungsgemäße Umsetzung benötigte tertiäre Amin III kann eine beliebige tertiäre Stickstoffbase sein, also z. B. ein Trialkylamin mit $C_1$-$C_{20}$-Alkylresten, wie Trimethylamin, Triethylamin, Tributylamin und Dimethylstearylamin, ein cycloaliphatisches Amin wie N-Methylpyrrolidin, N-Methylpiperidin und N-Methylmorpholin, ein aromatisches Amin wie Dimethylanilin other eine heterocyclische Stickstoffbase wie Pyridin oder Chinolin. Besonders geeignet sind polycyclische Brückenkopfamine wie 1,4-Diazabicyclo-[2.2.2]-octan (« DABCO »).

Es ist ein wesentliches Merkmal der Erfindung, daß das tertiäre Amin III nicht optisch aktiv zu sein braucht wie bei dem eingangs erwähnten Verfahren nach Pracejus, jedoch kann die optische Ausbeute in manchen Fällen noch erhöht werden, wenn man ein optisch aktives tertiäres Amin, z. B. ein optisch aktives 1,2-Dimethylpiperidin oder N,N-Dimethyl-1-phenylethylamin verwendet. Welches der beiden Enantiomeren die Reaktion im gewünschten Sinne beeinflußt (das andere kann sie u. U. beeinträchtigen), läßt sich durch einen einfachen Vorversuch ermitteln.

Da die erfindungsgemäße Reaktion vermutlich über eine Zwischenverbindung des Typs

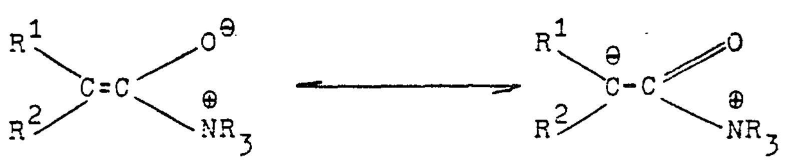

verläuft, die dem Proton des optisch aktiven Alkohols IV eine bevorzugte Eintrittsrichtung in das Molekül bietet, empfiehlt es sich, das tertiäre Amin III in ausreichender Menge einzusetzen, damit dieser Effekt nicht durch eine weniger spezifische Addition des Alkohols, wie sie bei Abwesenheit des Amins zu beobachten ist, überlagert wird. Im allgemeinen verwendet man daher 0,5-1 mol des Amins III pro Mol II, jedoch genügen häufig auch geringere Mengen (etwa bis herab zu 0,1 mol). In manchen Fällen kann auch ein stöchiometrischer Überschuß des Amins bis zu etwa 10 mol von Vorteil sein.

Geht man bei dem erfindungsgemäßen Verfahren nicht von den isolierten Ketenen II aus sondern läßt diese *in situ,* z. B. aus den Carbonsäurechloriden II', entstehen, so setzt man vorzugsweise die Gesamtmenge des für beide Reaktionsschritte benötigten Amins ein, also 1,1-10, vorzugsweise 1,5-2 mol pro Mol II'. Da man für die Ketenbildung in der Regel ein stark basisches Amin benötigt, für den erfindungsgemäßen Schritt hingegen bevorzugt eines, in welchem das N-Atom wie beim (relativ schwach basischen) Pyridin oder wie beim DABCO möglichst wenig sterisch behindert ist, empfiehlt es sich häufig auch, zwei verschiedene Amine zu verwenden.

Die Art des Alkohols IV, sofern dieser nur optisch aktiv ist, hat auf das Gelingen des erfindungsgemäßen Verfahrens nach den bisherigen Beobachtungen prinzipiell keinen Einfluß. Obgleich sich das Chiralitätszentrum grundsätzlich an beliebiger Stelle des Moleküls befinden kann, erzielt man die besten

Ergebnisse mit Alkoholen, in denen sich die Hydroxylgruppe an einem chiralen C-Atom befindet. Ferner ist es nicht von Relevanz, ob der Alkohol ein oder mehrere Chiralitätszentren enthält.

Geeignete asymmetrische Alkohole sind z. B. die optischen Isomeren von iso- und heterocyclischen 1-Aryl-$C_1$-$C_4$-alkan-1-olen, wobei die Arylgruppe u. a. durch $C_1$-$C_4$-Alkylgruppen, $C_1$-$C_4$-Alkoxygruppen, Halogen, Nitrogruppen, Cyangruppen, Dialkylaminogruppen und Alkylenaminogruppen wie die N-Pyrrolidino- und N-Piperidinogruppe substituiert sein kann. Bevorzugte Arylgruppen sind die Naphthylgruppe, die Pyridylgruppe und vor allem die Phenylgruppe. Der einfachste und daher häufig besonders bevorzugte Alkohol ist das 1-Phenylethan-1-ol. Weiterhin kommen optisch aktive Terpenalkohole wie das Menthol sowie optisch aktive Aminoalkohole IV' wie N-Methylephedrin (1-Phenyl-2-dimethylamino-propan-1-ol) und 2-Amino-butan-1-ol in Betracht. Bei Verwendung der Aminoalkohole kann sich die Mitverwendung eines gesonderten Amins III naturgemäß erübrigen.

Man setzt den Alkohol IV mindestens in stöchiometrischer, jedoch vorzugsweise in überschüssiger (bis etwa zu 10 mol) Menge zum Keten II ein.

Im übrigen nimmt man die Umsetzung wie eine übliche Alkoholaddition an Ketene vor. Für die Reaktionstemperaturen empfiehlt sich ein Bereich von (– 80) – 100 °C, wobei die stereospezifische Selektivität normalerweise mit steigender Temperatur abnimmt. Da man in der Regel jedoch bereits bei Raumtemperatur befriedigende optische Ausbeuten von über 60 % erzielt, lohnt es sich meistens nicht, zur relativ nur noch geringfügigen Erhöhung der optischen Ausbeute bei tieferen Temperaturen zu arbeiten.

Als Lösungsmittel eignen sich aprotische Flüssigkeiten wie Benzol, Toluol, $C_4$-$C_8$-Alkane, Cyclohexan, Ether wie Dimethyl- und Diethylether und cyclische Ether wie Dioxan und Tetrahydrofuran sowie Halogenalkane wie Methylenchlorid. Vorzugsweise arbeitet man in 1-20 %iger Lösung. Nach beendeter Umsetzung arbeitet man das Reaktionsgemisch wie üblich auf, indem man das Lösungsmittel, das überschüssige Amin III und den überschüssigen Alkohol von dem gebildeten Ester I'

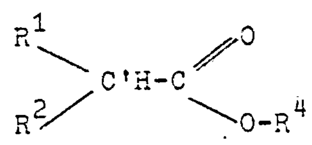

(I')

$R^4$ = Rest des Alkohols $R^4OH$ (IV) abdestilliert, wobei eine Fraktionierung dieser Komponenten, sofern sie für einen weiteren Reaktionsansatz wiederverwendet werden sollen, nicht erforderlich ist. Ist man vom Carbonsäurechlorid II' ausgegangen, so trennt man das hierbei meistens in kristalliner Form anfallende Ammoniumchlorid $R_3N \cdot HCl$ vor der weiteren Aufarbeitung ab.

Der Ester I' wird sodann in üblicher Weise — z. B. mit verdünnter Salzsäure oder mit verdünnter Natronlauge bei 30-100 °C in die Säure I bzw. das Na-Salz der Säure einerseits und den Alkohol IV gespalten, wonach der Alkohol wieder in die Reaktion zurückgeführt werden kann. Die Säure wird wie üblich isoliert und gewünschtenfalls gereinigt.

Da weder nennenswerte Mengen des Amıns III noch des Alkohols IV verbraucht werden, besteht das erfindungsgemäße Verfahren letztlich darin, ein (prochirales) Keten II bzw. dessen Vorstufe, ein racemisches Carbonsäurechlorid II', in einer hohen optischen Ausbeute in eine optisch aktive Carbonsäure I zu überführen. Hierbei ist es von Vorteil, daß sich das Verfahren offensichtlich besonders gut für eine kontinuierliche Arbeitsweise eignet.

In aller Regel lassen sich optische Ausbeuten von mindestens 30, meistens jedoch 70-80 %, erzielen. Welches der beiden Isomeren, ob das R- oder das S-Isomere hierbei im Überschuß gebildet wird, läßt sich zuverlässig nicht vorhersagen, jedoch kehrt sich das R/S-Verhältnis um, wenn man den antipodischen Alkohol einsetzt.

Die Verfahrensprodukte I sind entweder selber wichtige Wirkstoffe oder Zwischenprodukte für die Herstellung physiologisch aktiver Substanzen. Als leicht zugängliche optisch aktive Säuren dienen sie ihrerseits auch zur Trennung racemischer Alkohole oder Amine über diastereomere Verbindungspaare.

## Beispiel 1

Herstellung von R-Hydratropasäure (2-Phenylpropionsäure)

Eine Lösung aus 1,6 g (10 mmol) R,S-Hydratropasäurechlorid und 50 ml Toluol wurde bei 0 °C mit einer Lösung aus 1,0 g (10 mmol) Triethylamin und 25 ml Toluol versetzt, wonach diese Lösung noch 30 min bei 0 °C und 3 h bei 25 °C gerührt wurde. Hierbei färbte sich die Lösung gelb und es entstand ein weißer Niederschlag.

Dieses Gemisch wurde sodann bei 0 °C schnell mit einer Lösung aus 0,8 g (10 mmol) Pyridin und 1,6 g (10 mmol) 1-Menthol (2-Isopropyl-5-methyl-cyclohexanol) versetzt und danach noch 3 h bei 0 °C gehalten. Anschließend wurde das Gemisch 12 h lang bei 50 °C mit 50 ml konzentrierter Salzsäure gerührt, wonach die wäßrige Phase abgetrennt und die organische Phase wie üblich auf die Hydratropasäure aufgearbeitet wurde.

Die durch Kugelrohrdestillation gereinigte Säure (Ausbeute 78 %) zeigte einen Drehwert $[\alpha]_D^{22}$ (Aceton) von (– 36,0)°. Dies entspricht einer optischen Ausbeute von 38 % an der R-Hydratropasäure bzw. einem Enantiomerenverhältnis von 69 : 31.

## Beispiel 2

Herstellung von S-2-Phenoxypropionsäure

Diese Säure wurde analog Beispiel 1 aus R,S-2-Phenoxypropionylchlorid, Triethylamin, Pyridin und S-1-Phenylethanol in einer optischen Ausbeute von 28 % entsprechend einem Enantiomerenverhältnis von 64 : 36 hergestellt.

## Beispiel 3

Herstellung von S-Hydratropasäure

Eine Lösung aus 0,1 g (0,6 mmol) R,S-Hydratropasäurechlorid und 2 ml Toluol wurde bei 0 °C mit einer Lösung aus 0,6 mmol Triethylamin, 0,6 mmol Pyridin, 0,07 (0,6 mmol) S-1-Phenylethanol und 4 ml Toluol versetzt und analog Beispiel 1 auf die S-Hydratropasäure aufgearbeitet.

Die optische Ausbeute an der S-Hydratropasäure betrug 60 %. Bei Verwendung der gleichen molaren Menge 1,4-Diazabicyclo-[2,2,2]-octan (DABCO) anstelle des Triethylamins und des Pyridins als tertiärem Amin betrug die optische Ausbeute 72 %.

## Beispiel 4

Herstellung von R-Hydratropasäure

Analog Beispiel 3, jedoch mit R-1-Phenylethanol, fiel die R-Hydratropasäure bei Verwendung von Triethylamin und Pyridin in 72 %iger optischer Ausbeute und bei Verwendung von DABCO in 74 %iger optischer Ausbeute an.

## Beispiel 5

Herstellung von (+)-2-(6-Methoxynaphthyl-2)-propionsäure

Eine Lösung aus 0,3 g (1,2 mmol) racemischen 2-(6-Methoxynaphthyl-2)-propionylchlorid und 20 ml Toluol wurde bei 0 °C mit 0,12 g (1,2 mmol) Triethylamin versetzt, 3 h bei 25 °C gerührt und danach bei 0 °C mit einer Lösung aus 0,1 g (1,2 mmol) Pyridin, 0,15 g (1,2 mmol) S-1-Phenylethanol und 20 ml Toluol vereinigt und 2 h gerührt. Die weitere übliche Aufarbeitung auf die oben genannte Säure lieferte diese in einer optischen Ausbeute von 57 %.

## Beispiel 6

Herstellung von S-2-Chlor-phenylessigsäure

Diese Verbindung wurde analog Beispiel 5 aus 2-Chlor-phenyl-acetylchlorid und 1-Phenylethanol sowie mit Diethylether als Lösungsmittel in einer optischen Ausbeute von 47 % hergestellt.

## Beispiel 7

Herstellung von S-Hydratropasäure

1,32 g (10 mmol) Phenylmethylketen wurden mit 1,78 g (10 mmol) (+)-N-Methylpseudoephedrin (1-Phenyl-2-dimethylamino-propan-1-ol) in rd. 10 %iger toluolischer Lösung bei 70 °C miteinander umgesetzt, wonach das Reaktionsgemisch analog Beispiel 1 weiterverarbeitet wurde. Hierbei fiel die S-Hydratropasäure in einer optischen Ausbeute von 32 % an.

## Beispiel 8

Herstellung von S-Hydratropasäure

1,32 g (10 mmol) Phenylmethylketen wurden in rd. 10 %iger toluolischer Lösung in Gegenwart von 1,5 g (10 mmol) S-N-Dimethyl-1-phenylethylamin (opt. Reinheit 96 %) mit 1,22 g (10 mmol) S-1-Phenylethanol bei 0 °C umgesetzt und analog Beispiel 1 auf die Hydratropasäure aufgearbeitet. Die optische Ausbeute an der S-Hydratropasäure betrug 80 % entsprechend einem Enantiomerenverhältnis von 90 : 10.

Bei Verwendung des antipodischen Amins unter sonst gleichen Bedingungen betrug die optische Ausbeute an der S-Hydratropasäure 58 %, entsprechend einem Enantiomerenverhältnis von 79 : 21.

## Ansprüche

1. Verfahren zur Herstellung optisch aktiver Carbonsäuren der allgemeinen Formel I

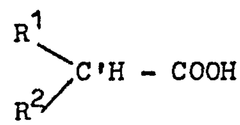 (I)

in welcher $R^1$ für einen organischen Rest steht, der über ein C-Atom mit dem asymmetrischen C'-Atom verknüpft ist, und in welcher $R^2$ einen der Reste $R^1$ (jedoch nicht den gleichen Rest), Halogen oder einen organischen Rest bedeutet, der über ein O-Atom an das asymmetrische C'-Atom gebunden ist, durch Umsetzung eines Ketens II

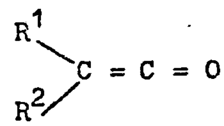 (II)

mit einem Alkohol in homogener flüssiger Phase in Gegenwart eines tertiären Amins III und anschließende Überführung des so erhaltenen Esters in die Säure, dadurch gekennzeichnet, daß man hierzu als Alkohol einen optisch aktiven Alkohol IV verwendet.

2. Abwandlung des Verfahrens gemäß Anspruch 1, dadurch gekennzeichnet, daß man anstelle des tertiären Amins III und des optisch aktiven Alkohols IV einen optisch aktiven tert.-Aminoalkohol IV' verwendet.

3. Verfahren nach den Ansprüchen 1 und 2, dadurch gekennzeichnet, daß man hierzu die Lösung eines Ketens II einsetzt, welches man *in situ* aus einem Carbonsäurechlorid II'

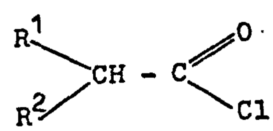 (II')

und einem tertiären Amin hergestellt hat.

4. Verfahren nach Anspruch 1, dadurch gekennzeichnet, daß man als tertiäres Amin ein optisch aktives tertiäres Amin verwendet.

## Claims

1. A process for the preparation of an optically active carboxylic acid of the formula I

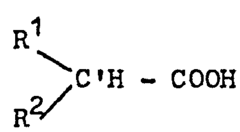 (I)

where $R^1$ is an organic radical which is linked to the asymmetric C' atom via a carbon atom, and $R^2$ is one of the radicals $R^1$ (but not the same radical), halogen or an organic radical which is bonded to the asymmetric C' atom via an oxygen atom, by reacting a ketene II

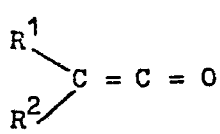 (II)

with an alcohol in a homogeneous liquid phase in the presence of a tertiary amine III and then converting the resulting ester into the acid, wherein an optically active alcohol IV is used as the alcohol.

2. A modification of the process as claimed in claim 1, wherein an optically active tert.-aminoalcohol IV' is used instead of the tertiary amine III and the optically active alcohol IV.

3. A process as claimed in claims 1 and 2, wherein a solution of a ketene II which has been prepared *in situ* from a carboxylic acid chloride II'

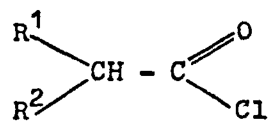

(II')

and a tertiary amine is used.

4. A process as claimed in claim 1, wherein an optically active tertiary amine is used as the tertiary amine.

**Revendications**

1. Procédé de préparation d'acides carboxyliques optiquement actifs de la formule générale I

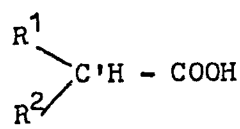

(I)

dans laquelle $R^1$ représente un reste organique, relié par un atome de carbone à l'atome de carbone asymétrique C', et $R^2$ possède la même signification que $R^1$, sans lui être identique, ou représente un atome d'halogène ou un reste organique, relié par un atome d'oxygène à l'atome de carbone asymétrique C', par réaction d'un cétène II

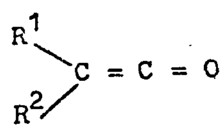

(II)

en phase liquide homogène et en présence d'une amine tertiaire III avec un alcool, suivie de la transformation de l'ester formé en acide, caractérisé en ce que l'alcool employé est un alcool optiquement actif IV.

2. Variante du procédé suivant la revendication 1, caractérisée en ce que l'amine tertiaire III et l'alcool optiquement actif IV sont remplacés par un tert.-amino-alcool IV' optiquement actif.

3. Procédé suivant les revendications 1 et 2, caractérisé en ce que l'on utilise la solution d'un cétène II, préparé *in situ* à partir d'un chlorure d'acide carboxylique II'

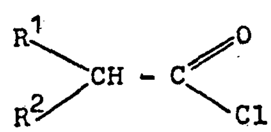

(II')

et d'une amine tertiaire.

4. Procédé suivant la revendication 1, caractérisé en ce que l'amine tertiaire mise en œuvre est une amine tertiaire optiquement active.